# EUROPEAN PATENT APPLICATION

(11) **EP 4 190 329 A1**
(43) Date of publication of application: **07.06.2023**
(21) Application number: 21850735.8
(22) Date of filing: 30.06.2021
(51) Int. Cl.: A61K 31/444, A61P 11/00, A61P 29/00, A61P 31/12, A61P 31/14, C12N 15/09

(54) **COLD REMEDY AND ANTIVIRAL AGENT**

(30) Priority: 30.07.2020 JP 2020128943
(71) Applicant: Otsuka Pharmaceutical Factory, Inc., Naruto-shi, Tokushima 772-8601 (JP)
(72) Inventor: IWATA, Koushi, Naruto-shi, Tokushima 772-8601 (JP); IMAI, Kaoru, Naruto-shi, Tokushima 772-8601 (JP)
(74) Representative: Hoffmann Eitle
(86) International application number: PCT/JP2021/024778
(87) International publication number: WO 2022/024649

(57) **Abstract**

Problem

To provide a novel cold medicine or antiviral agent.

Means for Solution

A cold medicine comprising a compound represented by formula (1) or a pharmaceutically acceptable salt thereof.

## Description

### Technical Field

Disclosed is an invention relating to a cold medicine and an antiviral agent.

### Background Art

Known colds include common colds generally referred to as "colds," and colds that are epidemic sometimes with serious symptoms, such as severe acute respiratory syndrome (SARS) and Middle East respiratory syndrome (MERS). Common colds are infections with four types of coronaviruses: HCoV-229E, HCoV-OC43, HCoV-NL63, and HCoV-HKU1. It is known that SARS and MERS are also caused by infections with coronaviruses called SARS-CoV and MERS-CoV, respectively. Infections with new types of viruses, such as SARS and MERS, sometimes cause a pandemic and have a major impact on humankind. In particular, in early 2020, infection with the so-called new coronavirus (COVID-19) has caused a pandemic, resulting in a large number of deaths and still causing great damage to humankind. Not limited to epidemic and severe colds, drugs that suppress symptoms, such as antipyretics and antitussives, are prescribed exclusively for the treatment thereof.

### Citation List

### Patent Literature

PTL 1: WO2017/110237

### Summary of Invention

### Technical Problem

An object is to provide a novel cold medicine or antiviral agent.

### Solution to Problem

Through intensive research to achieve the objects including the above object, it was found that the compound disclosed in PTL 1 as a compound having an LPL activating action has antiviral activity. Based on this finding and further studies, the invention represented by the following is provided.

### Item 1.

A cold medicine comprising a compound represented by the following formula (1) or a pharmaceutically acceptable salt thereof: 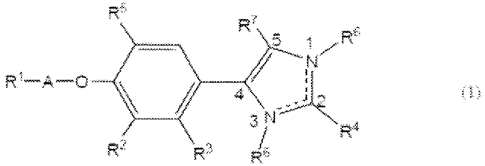 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

### Item 2.

The cold medicine according to Item 1, wherein R⁴ is 6-halogenopyridin-3-yl.

### Item 3.

The cold medicine according to Item 1 or 2, wherein R¹ is pyridin-2-yl having halogen-substituted C₁-C₆ alkyl at the 3-position and halogen at the 5-position.

### Item 4.

The cold medicine according to any one of Items 1 to 3, wherein A is methylene.

### Item 5.

The cold medicine according to any one of Items 1 to 4, wherein R¹ is 3-fluoro-5-(trifluoromethyl)pyridin-2-yl, and R⁴ is 6-fluoropyridin-3-yl.

### Item 6.

The cold medicine according to any one of Items 1 to 5, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine Item 7.

An antiviral agent comprising a compound represented by the following formula (1) or a pharmaceutically acceptable salt thereof: 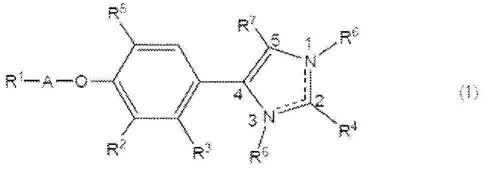 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

### Item 8.

The antiviral agent according to Item 7, wherein R⁴ is 6-halogenopyridin-3-yl.

### Item 9.

The antiviral agent according to Item 7 or 8, wherein R¹ is pyridin-2-yl having halogen-substituted C₁-C₆ alkyl at the 3-position and halogen at the 5-position.

### Item 10.

The antiviral agent according to any one of Items 7 to 9, wherein A is methylene.

### Item 11.

The antiviral agent according to any one of Items 7 to 10, wherein R¹ is 3-fluoro-5-(trifluoromethyl)pyridin-2-yl, and R⁴ is 6-fluoropyridin-3-yl.

### Item 12.

The antiviral agent according to any one of Items 7 to 11, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine Item 13.

The antiviral agent according to any one of Items 7 to 12, wherein the virus is coronavirus.

### Item A1.

A method for treating cold, comprising administering a compound represented by the following formula (1) or a pharmaceutically acceptable salt thereof to a patient in need thereof: 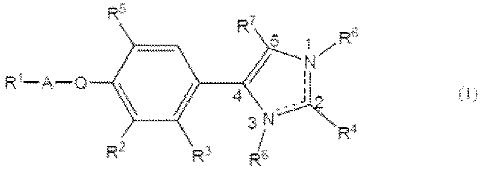 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

### Item A2.

The method according to Item A1, wherein R⁴ is 6-halogenopyridin-3-yl.

### Item A3.

The method according to Item A1 or A2, wherein R¹ is pyridin-2-yl having halogen-substituted C₁-C₆ alkyl at the 3-position and halogen at the 5-position.

### Item A4.

The method according to any one of Items A1 to A3, wherein A is methylene.

### Item A5.

The method according to any one of Items A1 to A4, wherein R¹ is 3-fluoro-5-(trifluoromethyl)pyridin-2-yl, and R⁴ is 6-fluoropyridin-3-yl.

### Item A6.

The method according to any one of Items A1 to A5, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine Item A7.

A method for treating an infection caused by a virus, comprising administering a compound represented by the following formula (1) or a pharmaceutically acceptable salt thereof to a patient in need thereof: 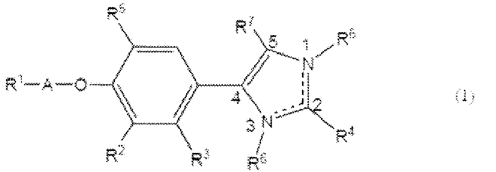 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

### Item A8.

The method according to Item A7, wherein R⁴ is 6-halogenopyridin-3-yl.

### Item A9.

The method according to Item A7 or A8, wherein R¹ is pyridin-2-yl having halogen-substituted C₁-C₆ alkyl at the 3-position and halogen at the 5-position.

### Item A10.

The method according to any one of Items A7 to A9, wherein A is methylene.

### Item A11.

The method according to any one of Items A7 to A10, wherein R¹ is 3-fluoro-5-(trifluoromethyl)pyridin-2-yl, and R⁴ is 6-fluoropyridin-3-yl.

### Item A12.

The method according to any one of Items A7 to A11, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine Item A13.

The method according to any one of Items A7 to A12, wherein the virus is coronavirus.

### Item B1.

Use of a compound represented by the following formula (1) or a pharmaceutically acceptable salt thereof for producing a cold medicine: 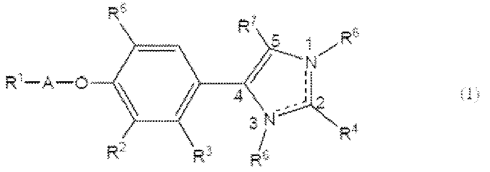 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

### Item B2.

The use according to Item B1, wherein R⁴ is 6-halogenopyridin-3-yl.

### Item B3.

The use according to Item B1 or B2, wherein R¹ is pyridin-2-yl having halogen-substituted C₁-C₆ alkyl at the 3-position and halogen at the 5-position.

### Item B4.

The use according to any one of Items B1 to B3, wherein A is methylene.

### Item B5.

The use according to any one of Items B1 to B4, wherein R¹ is 3-fluoro-5-(trifluoromethyl)pyridin-2-yl, and R⁴ is 6-fluoropyridin-3-yl.

### Item B6.

The use according to any one of Items B1 to B5, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine Item B7.

Use of a compound represented by the following formula (1) or a pharmaceutically acceptable salt thereof for producing an antiviral agent: 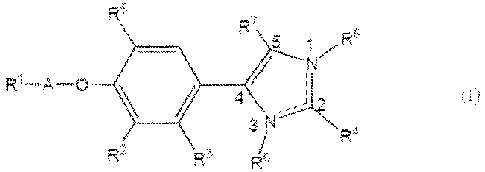 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

### Item B8.

The use according to Item B7, wherein R⁴ is 6-halogenopyridin-3-yl.

### Item B9.

The use according to Item B7 or B8, wherein R¹ is pyridin-2-yl having halogen-substituted C₁-C₆ alkyl at the 3-position and halogen at the 5-position.

### Item B10.

The use according to any one of Items B7 to B9, wherein A is methylene.

### Item B11.

The use according to any one of Items B7 to B10, wherein R¹ is 3-fluoro-5-(trifluoromethyl)pyridin-2-yl, and R⁴ is 6-fluoropyridin-3-yl.

### Item B12.

The use according to any one of Items B7 to B11, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine Item B13.

The use according to any one of Items B7 to B12, wherein the virus is coronavirus.

### Advantageous Effects of Invention

A novel means effective for the treatment of cold is provided.

### Brief Description of Drawings

Fig. 1 shows the structures of compounds subjected to pharmacological tests in the Examples.
Fig. 2 shows the results of Pharmacological Test 1 in the Examples.
Fig. 3 shows the results of Pharmacological Test 2 in the Examples.
Fig. 4 shows the results of Pharmacological Test 3 in the Examples.

### Description of Embodiments

### 1. Compound of Formula (1)

Examples of halogen atoms include fluorine, chlorine, bromine, iodine, and the like.

Examples of C₁-C₆ alkyl groups include linear or branched alkyl groups having 1 to 6 carbon atoms, such as methyl, ethyl, n-propyl, n-butyl, n-pentyl, n-hexyl, 1-methylethyl, tert-butyl, and 2-methylbutyl.

Examples of C₁-C₆ alkoxy groups include linear or branched alkoxy groups having 1 to 6 carbon atoms, such as methoxy, ethoxy, n-propoxy, n-butoxy, n-pentyloxy, n-hexyloxy, 1-methylethoxy, tert-butoxy, and 2-methylbutoxy.

Examples of C₁-C₆ alkylene groups include linear or branched alkylene groups having 1 to 6 carbon atoms, such as methylene, ethylene, ethylidene, trimethylene, tetramethylene, pentamethylene, and hexamethylene.

Examples of halogen-substituted C₁-C₆ alkyl groups include halogenoalkyl groups that are linear or branched C₁-₆ alkyl groups substituted with one or more halogen atoms selected from the group consisting of fluorine, chlorine, bromine, and iodine. A preferable example of halogen-substituted C₁-C₆ alkyl groups is a perhalogenoalkyl group, and a more preferable example is a perfluoroalkyl group. Specific examples of halogen-substituted C₁-C₆ alkyl groups include trifluoromethyl, pentafluoroethyl, heptafluoropropyl, nonafluorobutyl, undecafluoropentyl, tridecafluorohexyl, 2,2,2-trifluoroethyl, 3,3,3-trifluoropropyl, 4,4,4-trifluorobutyl, 5,5,5-trifluoropentyl, 6,6,6-trifluorohexyl, and the like.

Examples of pyridyl groups having two substituents selected from the group consisting of halogen and halogen-substituted C₁-C₆ alkyl include 3,4-ditrifluoromethyl-2-pyridyl, 3,5-ditrifluoromethyl-2-pyridyl, 3,6-ditrifluoromethyl-2-pyridyl, 2,4-ditrifluoromethyl-3-pyridyl, 2,5-ditrifluoromethyl-3-pyridyl, 2,6-ditrifluoromethyl-3-pyridyl, 2,3-ditrifluoromethyl-3-pyridyl, 3,5-dipentafluoroethyl-2-pyridyl, 3,5-diheptafluoropropyl-2-pyridyl, 3,5-dinonafluorobutyl-2-pyridyl, 3,5-diundecafluoropentyl-2-pyridyl, 3,5-ditridecafluorohexyl-2-pyridyl, 5-bromo-3-fluoro-2-pyridyl, 5-chloro-3-fluoro-2-pyridyl, 3-fluoro-5-iodo-2-pyridyl, 5-bromo-3-chloro-2-pyridyl, 5-bromo-3-iodo-2-pyridyl, 3-bromo-5-fluoro-2-pyridyl, 3-chloro-5-fluoro-2-pyridyl, 5-fluoro-3-iodo-2-pyridyl, 3-bromo-5-chloro-2-pyridyl, 3-bromo-5-iodo-2-pyridyl, 3-fluoro-5-trifluoromethyl-2-pyridyl, 5-fluoro-3-trifluoromethyl-2-pyridyl, 3-chloro-5-trifluoromethyl-2-pyridyl, 3-bromo-5-trifluoromethyl-2-pyridyl, 3-iodo-5-trifluoromethyl-2-pyridyl, 3-fluoro-5-pentafluoroethyl-2-pyridyl, 3-fluoro-5-heptafluoropropyl-2-pyridyl, 3-fluoro-5-nonafluorobutyl-2-pyridyl, 3-fluoro-5-undecafluoropentyl-2-pyridyl, 3-fluoro-5-tridecafluorohexyl-2-pyridyl, and the like. As such, the two substituents selected from the group consisting of halogen and halogen-substituted C₁-C₆ alkyl may be the same or different.

In an embodiment, preferable pyridyl groups having two substituents selected from the group consisting of halogen and halogen-substituted C₁-C₆ alkyl are pyridin-2-yl groups having halogen-substituted C₁-C₆ alkyl at the 3-position and halogen at the 5-position. Preferable examples include 3-fluoro-5-trifluoromethyl-2-pyridyl, 3-bromo-5-trifluoromethyl-2-pyridyl, 3-fluoro-5-pentafluoroethyl-2-pyridyl, 3-fluoro-5-heptafluoropropyl-2-pyridyl, 3-fluoro-5-nonafluorobutyl-2-pyridyl, 3-fluoro-5-undecafluoropentyl-2-pyridyl, 3-fluoro-5-tridecafluorohexyl-2-pyridyl, and the like; and a more preferable example is 3-fluoro-5-trifluoromethyl-2-pyridyl.

Examples of pyridyl groups substituted with halogen include 3-fluoro-2-pyridyl, 4-fluoro-2-pyridyl, 5-fluoro-2-pyridyl, 6-fluoro-2-pyridyl, 2-fluoro-3-pyridyl, 4-fluoro-3-pyridyl, 5-fluoro-3-pyridyl, 6-fluoro-3-pyridyl, 2-fluoro-4-pyridyl, 3-fluoro-4-pyridyl, 6-chloro-3-pyridyl, 6-bromo-3-pyridyl, 6-iodo-3-pyridyl, and the like. Among these, preferred are 6-halogenopyridin-3-yl groups, such as 6-fluoro-3-pyridyl, 6-chloro-3-pyridyl, 6-bromo-3-pyridyl, and 6-iodo-3-pyridyl; and more preferred is 6-fluoro-3-pyridyl.

In an embodiment, as shown in (1-1) below, the bond between N at the 3-position and C at the 2-position of the imidazole skeleton in formula (1) is a double bond, and the bond between C at the 2-position and N at the 1-position of the imidazole skeleton in formula (1) is a single bond. In another embodiment, as shown in (1-2) below, the bond between N at the 3-position and C at the 2-position of the imidazole skeleton in formula (1) is a single bond, and the bond between C at the 2-position and N at the 1-position of the imidazole skeleton in formula (1) is a double bond.

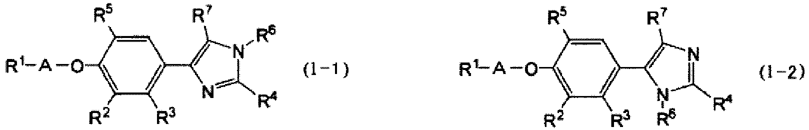

Preferable examples of compounds represented by formula (1) include the following compounds:
- 2-[[4-(5-bromo-2-(6-fluoropyridin-3-yl)-1H-imidazol-4-yl)-3-methoxyphenoxy]methyl]-3-fluoro-5-(trifluoromethyl)pyridine
- 2-[[4-(5-chloro-2-(6-fluoropyridin-3-yl)-1H-imidazol-4-yl)-3-methoxyphenoxy]methyl]-3-fluoro-5-(trifluoromethyl)pyridine
- 2-[[3-chloro-4-(5-chloro-2-(6-fluoropyridin-3-yl)-1H-imidazol-4-yl)phenoxy]methyl]-3-fluoro-5-(trifluoromethyl)pyridine

The compound represented by formula (1) may be a pharmaceutically acceptable salt thereof. The pharmaceutically acceptable salt is not particularly limited and, for example, can be at least one member selected from the group consisting of hydrochloride, nitrate, sulfate, hydrobromide, phosphate, carbonate, sulfonate, acetate, lactate, and citrate. These acid addition salts can be produced according to usual methods.

The compound represented by formula (1) may include optical isomers having a carbon atom as an asymmetric center. The compound represented by formula (1) includes all racemates that are mixtures of such optical isomers, and optically active forms (i.e., optical isomers). The optical isomers can be separated using various known separation methods.

### 2. Production method

The compound represented by formula (1) can be produced according to the production method disclosed in PTL 1.

### 3. Pharmaceutical preparation and administration route

The compound represented by formula (1) or a pharmaceutically acceptable salt thereof has excellent anti-infective and anti-proliferative activity against viruses. Therefore, the compound represented by formula (1) or a pharmaceutically acceptable salt thereof, or a pharmaceutical composition containing the same can be used as a cold medicine, an antiviral agent, a viral infection inhibitor, or a viral infection preventive or therapeutic agent.

The types of virus and viral infection are not particularly limited. Examples of viral infections include gastrointestinal infectious viral infections (e.g., enterovirus and cytomegalovirus), respiratory tract infectious viral infections (e.g., infections caused by respiratory tract infectious viruses, such as influenza virus, rhinovirus, coronavirus, parainfluenza virus, RS virus, adenovirus, and reovirus), shingles caused by herpesvirus, diarrhea caused by rotavirus, viral hepatitis, AIDS, and the like. In an embodiment, preferable viruses are coronaviruses (including SARS-CoV-2).

The present invention provides a pharmaceutical composition containing the compound represented by formula (1) or a pharmaceutically acceptable salt thereof. The pharmaceutical composition can be in the form of a general pharmaceutical preparation. The pharmaceutical composition may contain any pharmaceutically acceptable carriers in addition to the compound represented by formula (1) or a pharmaceutically acceptable salt thereof. Examples of pharmaceutically acceptable carriers include fillers, extenders, binders, humectants, disintegrators, surfactants, lubricants, diluents, and the like. These carriers can be suitably selected according to the unit dosage form of the resulting pharmaceutical compositions.

A variety of unit dosage forms can be selected for the pharmaceutical preparation mentioned above, depending on the therapeutic purpose. Typical examples include tablets, pills, powders, solutions, suspensions, emulsions, granules, capsules, suppositories, injections (solutions, suspensions, etc.), inhalers, ointments, and the like.

To form tablets, at least one member selected from the group consisting of the following carriers, for example, may be used as the pharmaceutically acceptable carrier mentioned above: lactose, sucrose, sodium chloride, glucose, urea, starch, calcium carbonate, kaolin, crystalline cellulose, silicic acid, potassium phosphate, and like excipients; water, ethanol, propanol, simple syrup, glucose solutions, starch solutions, gelatin solutions, carboxymethylcellulose, hydroxypropyl cellulose, methylcellulose, polyvinylpyrrolidone, and like binders; carboxymethylcellulose sodium, carboxymethylcellulose calcium, low-substituted hydroxypropyl cellulose, dry starch, sodium alginate, agar powder, laminarin powder, sodium hydrogen carbonate, calcium carbonate, and like disintegrators; polyoxyethylene sorbitan fatty acid esters, sodium lauryl sulfate, stearic acid monoglyceride, and like surfactants; sucrose, stearin, cacao butter, hydrogenated oils, and like disintegration inhibitors; quaternary ammonium bases, sodium lauryl sulfate, and like absorption promoters; glycerin, starch, and like humectants; starch, lactose, kaolin, bentonite, colloidal silicic acid, and like adsorbents; purified talc, stearate, boric acid powder, polyethylene glycol, and like lubricants; and the like. Further, such tablets may be coated with typical coating materials as required, to prepare, for example, sugar-coated tablets, gelatin-coated tablets, enteric-coated tablets, film-coated tablets, and double- or multi-layered tablets.

To form pills, at least one member selected from the group consisting of the following carriers, for example, may be used as the pharmaceutically acceptable carrier: glucose, lactose, starch, cacao butter, hydrogenated vegetable oils, kaolin, talc and like excipients; gum arabic powder, tragacanth powder, gelatin, ethanol, and like binders; laminarin, agar and like disintegrators; and the like.

To form suppositories, at least one member selected from the group consisting of the following carriers, for example, may be used as the pharmaceutically acceptable carrier: polyethylene glycol, cacao butter, higher alcohols, esters of higher alcohols, gelatin, semi-synthetic glycerides, and the like.

Capsules are prepared according to usual methods, by mixing the compound represented by formula (1) or a pharmaceutically acceptable salt thereof with the pharmaceutically acceptable carriers mentioned above and loading the mixture into a hard gelatin capsule, soft gelatin capsule, or the like.

To prepare injections such as solutions, emulsions, or suspensions, the injections are sterilized and preferably made isotonic to blood. To form such injections, at least one member selected from the group consisting of the following, for example, may be used as a diluent: water, ethanol, macrogol, propylene glycol, ethoxylated isostearyl alcohol, polyoxylated isostearyl alcohol, polyoxyethylene sorbitan fatty acid esters, and the like. In this case, the pharmaceutical preparation may contain sodium chloride, glucose, or glycerin in an amount sufficient to prepare an isotonic solution, and may also contain typical solubilizers, buffers, soothing agents, etc.

To form ointments such as pastes, creams, or gels, at least one member selected from the group consisting of the following, for example, may be used as a diluent: white petrolatum, paraffin, glycerin, cellulose derivatives, polyethylene glycol, silicone, bentonite, and the like.

The pharmaceutical composition may contain, if necessary, coloring agents, preservatives, fragrances, flavors, sweetening agents, etc., and/or other medicines.

The pharmaceutical composition preferably contains a therapeutically effective amount of the compound represented by formula (1) or a pharmaceutically acceptable salt thereof. The pharmaceutical composition may contain the compound represented by formula (1) or a pharmaceutically acceptable salt thereof, for example, in an amount of about 0.5 to 90 wt.%, and preferably about 1 to 85 wt.%.

The route of administration of the pharmaceutical composition is not particularly limited, and is determined by, for example, the form of the preparation, the patient's age and sex, the severity of the disease, and other conditions. For example, tablets, pills, solutions, suspensions, emulsions, granules, and capsules are orally administered. Injections are administered intravenously, intramuscularly, intracutaneously, subcutaneously, or intraperitoneally, singly or as mixed with usual injection transfusions, such as glucose solutions or amino acid solutions. Suppositories are administered intrarectally.

The dosage of the pharmaceutical composition is suitably selected according to the method of use, the patient's age, sex and other conditions, the severity of the disease, etc. For example, the amount of the compound represented by formula (1) or a pharmaceutically acceptable salt thereof can be about 0.5 to 20 mg, and preferably about 1 to 10 mg, per kg body weight per human adult per day. The pharmaceutical composition can be administered once a day, or in two to four portions a day.

### Examples

The present invention is described in more detail below with reference to Examples, but is not limited to these Examples.

### Preparation Example 1: Preparation of tablet

Using the compound of Example 281 disclosed in WO2017/110237 (2-[[4-(5-chloro-2-(6-fluoropyridin-3-yl)-1H-imidazol-4-yl)-3-methoxyphenoxy]methyl]-3-fluoro-5-(trifluoromethyl)pyridine, which is referred to below as "compound X") as an active ingredient, tablets (10000 tablets) each containing 300 mg of the compound were prepared according to the following formulation. All of the components used were products of Japanese Pharmacopoeia.

| | |
|---|---|
| Compound X | 3000 g |
| Lactose | 335 g |
| Cornstarch | 165 g |
| Carboxymethylcellulose calcium | 125 g |
| Methylcellulose | 60 g |
| Magnesium stearate | 15 g |

According to the above formulation, compound X, lactose, cornstarch, and carboxymethylcellulose calcium were sufficiently mixed. The mixture was granulated using an aqueous methylcellulose solution, screened with a 24-mesh screen, mixed with magnesium stearate, and pressed into tablets, thereby yielding the desired tablets.

### Preparation Example 2: Preparation of capsule

Using compound X as an active ingredient, hard gelatin capsules (10000 capsules) each containing 200 mg of compound X were prepared according to the following formulation. All of the components used were products of Japanese Pharmacopoeia.

| | |
|---|---|
| Compound X | 2000 g |
| Crystalline cellulose | 300 g |
| Cornstarch | 170 g |
| Talc | 20 g |
| Magnesium stearate | 10 g |

According to the above formulation, each of the components was ground into a fine powder, and the powders were mixed to form a uniform mixture and loaded into gelatin capsules of a desired size for oral administration, thereby yielding the desired capsules.

### Pharmacological Test 1

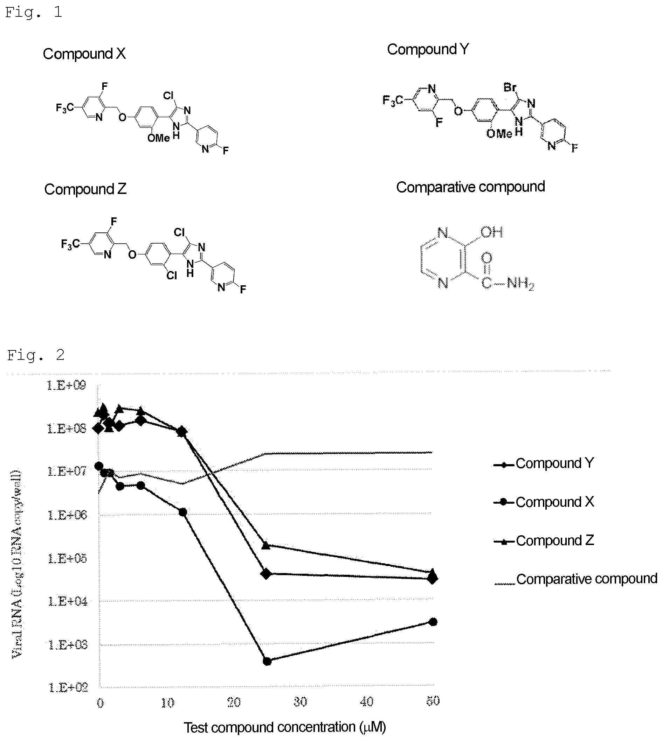

Compound X, and the compounds of Examples 255 and 361 disclosed in WO2017/110237 (referred to below as "compound Y" and "compound Z," respectively) were used as test compounds. 3-Hydroxypyrazine-2-carboxamide, which is reported to have antiviral activity (Transboundary Emerging Dis., 63, e205 (2016)) and is a compound related to favipiravir (commercially available as Avigan), was used as a comparative compound. Fig. 1 shows their chemical formulas.

MRC-5 (distributed from JCRB Cell Bank) was used as a host cell, and this was cultured on a 96-well plate until a monolayer sheet was obtained. Next, the medium was removed, 50 µL of a mixed suspension medium (medium: Dulbecco's Modified Eagle Medium) of a human coronavirus HCoV-229E solution (distributed from ATCC) and a predetermined dose of the test compound was added, and the resultant was allowed to stand at room temperature for 1 hour. The virus concentration was 3.5 TCID 50/mL as a logarithmic value. Subsequently, the medium was removed, and the cells were washed with phosphate buffered saline (calcium- and magnesium-free). Then, a new medium (containing 10% fetal bovine serum (FBS)) mixed with the same dose of the test compound as before was added, and the cells were cultured at 33°C in the presence of 5% carbon dioxide until a cytopathic effect appeared. After culturing (3 to 5 days), freeze-thawing was repeated 3 times, and RNA was extracted from the medium and quantified by reverse transcription reaction and qPCR. The conditions are as shown in the following tables.

**Table 1**

| Reverse transcription reaction conditions | | |
|---|---|---|
| Step | Reaction temperature (°C) | Time (min) |
| 1 | 25 | 10 |
| 2 | 37 | 120 |
| 3 | 85 | 5 |
| 4 | 4 | ∞ |

**Table 2**

| Primers and probe | | | |
|---|---|---|---|
| | Base sequence | SEQ. ID. No | Chain length |
| Forward primer | CAGTCAAATGGGCTGATGCA | 1 | 20 |
| Reverse primer | AAAGGGCTATAAAGAGAATAAGGTATTCT | 2 | 29 |
| Probe | FAM-CCCTGACGACCACGTTGTGGTTCA-BHQ1 | 3 | 24 |

**Table 3**

| qPCR reaction mixture | | |
|---|---|---|
| Reagent | Final concentration | Amount/tube |
| Forward primer | 400 nM | 0.8 µL |
| Reverse primer | 400 nM | 0.8 µL |
| Probe | 300 nM | 0.6 µL |
| qPCR Master Mix | | 10 µL |
| Nuclease-Free Water | | 2.8 µL |
| Reverse transcription reaction solution | | 5 µL |
| Total | | 20 µL |

**Table 4**

| qPCR reaction conditions | | | |
|---|---|---|---|
| Step | | Temperature (°C) | Time |
| 1 | Hot start | 95 | 10 min |
| 2 (40 cycles) | Modification | 95 | 8 sec |
| | Annealing and extension | 60 | 34 sec |

The results shown in the following table and Fig. 2 revealed that the compounds of the present invention, typified by compounds X to Z, showed a significantly higher coronavirus infection-suppressing effect than the comparative compound.

**Table 5**

| Test substance | Concentration (µM) 0 | Virus amount 1.E+08 | Test substance | Concentration (µM) 0 | Virus amount 1.E+07 | Test substance | Concentration (µM) 0 | Virus amount 2.E+08 | Test substance | Concentration (µM) 0 | Virus amount 3.E+06 |
|---|---|---|---|---|---|---|---|---|---|---|---|
| Compound Y | 0.78 | 2.E+08 | Compound X | 0.78 | 9.E+06 | Compound Z | 0.78 | 3.E+08 | Comparative compound | 1.6 | 1.E+07 |
| | 1.6 | 1.E+08 | | 1.58 | 1.E+07 | | 1.58 | 1.E+08 | | 3.1 | 7.E+06 |
| | 3.1 | 1.E+08 | | 3.1 | 5.E+06 | | 3.1 | 3.E+08 | | 6.3 | 9.E+06 |
| | 6.2 | 1.E+08 | | 6.3 | 5.E+06 | | 6.3 | 3.E+08 | | 12.5 | 5.E+06 |
| | 12.5 | 8.E+07 | | 12.5 | 1.E+06 | | 12.5 | 8.E+07 | | 25 | 2.E+07 |
| | 25 | 4.E+04 | | 25 | 4.E+02 | | 25 | 2.E+05 | | 50 | 2.E+07 |
| | 50 | 3.E+04 | | 50 | 3.E+03 | | 50 | 4.E+04 | | 100 | 2.E+07 |

| | | | | | | | | | | | |
|---|---|---|---|---|---|---|---|---|---|---|---|
| In the table, "1.E+08" means the 8th power of 1 × 10. | | | | | | | | | | | |

### Pharmacological Test 2

Compound X described above was used as the test compound. Comparative Compound 1 used herein was favipiravir, which has been approved as an anti-influenza drug in Japan and has been suggested to be also effective for the new coronavirus (SARS-CoV-2). Comparative Compound 2 used herein was chloroquine phosphate, which has temporarily been approved by the US Food and Drug Administration (FDA) for emergency use in patients with new coronavirus infection.

A test was conducted in the same manner as in Pharmacological Test 1 described above, except that HCT-8 (ATCC CCL-244) was used as the host cell, OC43 (ATCC VR1588) was used as the human coronavirus, the medium for first standing at room temperature for 1 hour did not contain the test compound, and the medium containing the test compound to be used later did not contain fetal bovine serum. The conditions for reverse transcription reaction and qPCR are as shown in the following tables.

**Table 6**

| Reverse transcription reaction conditions | | |
|---|---|---|
| Step | Reaction temperature (°C) | Time (min) |
| 1 | 25 | 10 |
| 2 | 37 | 120 |
| 3 | 85 | 5 |
| 4 | 4 | ∞ |

**Table 7**

| Primers and probe | | | |
|---|---|---|---|
| | Base sequence | SEQ. ID. No. | Chain length |
| Forward primer | CGATGAGGCTATTCCGACTAGGT | 4 | 23 |
| Reverse primer | CCTTCCTGAGCCTTCAATATAGTAACC | 5 | 27 |
| Probe | FAM-TCCGCCTGGCACGGTACTCCCT-BHQ1 | 6 | 22 |

**Table 8**

| qPCR reaction mixture | | |
|---|---|---|
| Reagent | Final concentration | Amount/tube |
| Forward primer | 400 nM | 0.8 µL |
| Reverse primer | 400 nM | 0.8 µL |
| Probe | 300 nM | 0.6 µL |
| qPCR Master Mix | | 10 µL |
| Nuclease-Free Water | | 2.8 µL |
| Reverse transcription reaction solution | | 5 µL |
| Total | | 20 µL |

**Table 9**

| qPCR reaction conditions | | | |
|---|---|---|---|
| Step | | Temperature (°C) | Time |
| 1 | Hot start | 95 | 10 min |
| 2 (40 cycles) | Modification | 95 | 3 sec |
| | Annealing | 55 | 10 sec |
| | Extension | 65 | 60 sec |

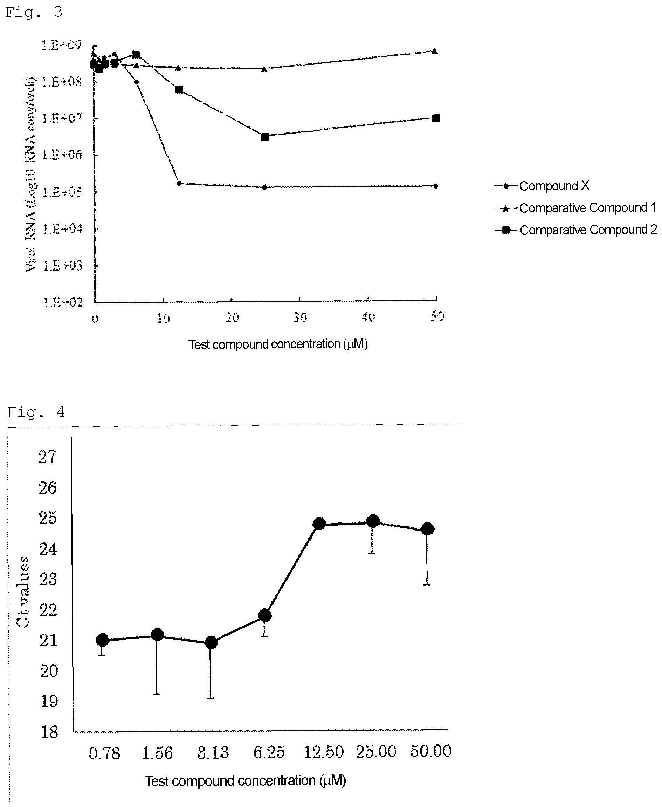

The results shown in the table below and Fig. 3 revealed that the compound of the present invention showed a significantly higher coronavirus infection-suppressing effect than both comparative compounds.

**Table 10**

| Test compound | Concentration (µM) | Virus amount | Test compound | Concentration (µM) | Virus amount | Test compound | Concentration (µM) | Virus amount |
|---|---|---|---|---|---|---|---|---|
| Compound X | 0 | 4.E+08 | Comparative compound 1 | 0 | 6.E+08 | Comparative compound 2 | 0 | 3.E+08 |
| | 0.78 | 3.E+08 | | 0.78 | 4.E+08 | | 0.78 | 2.E+08 |
| | 1.6 | 5.E+08 | | 1.58 | 3.E+08 | | 1.58 | 3.E+08 |
| | 3.1 | 6.E+08 | | 3.1 | 3.E+08 | | 3.1 | 4.E+08 |
| | 6.2 | 1.E+08 | | 6.3 | 3.E+08 | | 6.3 | 6.E+08 |
| | 12.5 | 2.E+05 | | 12.5 | 2.E+08 | | 12.5 | 6.E+07 |
| | 25 | 1.E+05 | | 25 | 2.E+08 | | 25 | 3.E+06 |
| | 50 | 1.E+05 | | 50 | 6.E+08 | | 50 | 9.E+06 |

| | | | | | | | | |
|---|---|---|---|---|---|---|---|---|
| In the table, "1.E+08" means the 8th power of 1 × 10. The same applies to other descriptions. | | | | | | | | |

### Pharmacological Test 3

Compound X described above was used as the test compound. Vero E6 (ATCC CRL1586) was used as a host cell, and this was cultured on a 96-well plate until a monolayer sheet was obtained. Next, the medium was removed, 50 µL of a mixed suspension medium of SARS-CoV-2 (hCoV-19/Germany/BY-ChVir-929/2020) and a predetermined dose of the test compound was added, and the resultant was allowed to stand at 37°C for 1 hour. The virus concentration was 150 PFU/well. Subsequently, the medium was removed, and the cells were washed with PBS. Then, a new medium mixed with the same dose of the test compound as before was added, and the cells were cultured at 37°C overnight. After culturing, the virus amount in the culture supernatant was quantified by qPCR. As the primers and probe, the new coronavirus N1 protein-specific primers and probe specified by the Centers for Disease Control and Prevention (CDC) were used, and the conditions were also in accordance with the conditions specified by the CDC. The number of PCR cycles (Ct value) until the signal intensity of the PCR product reached a predetermined intensity (threshold value) was determined.

The results shown in the table below and Fig. 4 revealed that the compounds of the present invention, typified by compound X, also showed an infection-suppressing effect on SARS-CoV-2, which is a new type of coronavirus.

| Test compound | µM | Ct value (average value) |
|---|---|---|
| Compound X | 0.78 | 21.0 |
| | 1.56 | 21.1 |
| | 3.13 | 20.9 |
| | 6.25 | 21.7 |
| | 12.50 | 24.7 |
| | 25.00 | 24.8 |
| | 50.00 | 24.5 |

## Claims

1. A cold medicine comprising a compound represented by the following formula (1) or a pharmaceutically acceptable salt thereof: 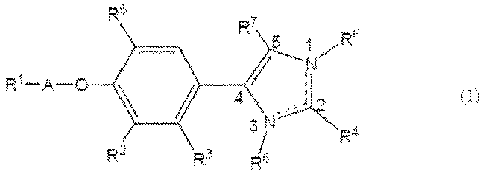 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

2. The cold medicine according to claim 1, wherein R⁴ is 6-halogenopyridin-3-yl.

3. The cold medicine according to claim 1 or 2, wherein R¹ is pyridin-2-yl having halogen-substituted C₁-C₆ alkyl at the 3-position and halogen at the 5-position.

4. The cold medicine according to any one of claims 1 to 3, wherein A is methylene.

5. The cold medicine according to any one of claims 1 to 4, wherein R¹ is 3-fluoro-5-(trifluoromethyl)pyridin-2-yl, and R⁴ is 6-fluoropyridin-3-yl.

6. The cold medicine according to any one of claims 1 to 5, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine

7. An antiviral agent comprising a compound represented by the following formula (1) or a pharmaceutically acceptable salt thereof: 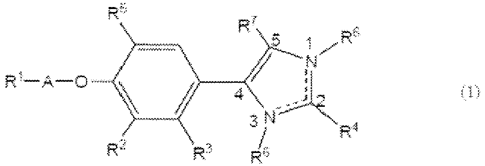 wherein R¹ is pyridyl having two substituents selected from the group consisting of halogen and halogen-substituted C₁-C₆ alkyl;
R², R⁵, and R⁶ are hydrogen;
R³ is C₁-C₆ alkoxy or halogen;
R⁴ is pyridyl substituted with halogen;
R⁶ is attached to only one of N at the 1-position and N at the 3-position of the imidazole skeleton, wherein R⁶ is attached to N at the 1-position when the bond between N at the 3-position and C at the 2-position of the imidazole skeleton is a double bond, and R⁶ is attached to N at the 3-position when the bond between N at the 3-position and C at the 2-position is a single bond;
R⁷ is halogen; and
A is C₁-C₆ alkylene,
wherein the bond between C at the 2-position and N at the 1-position is a single bond when the bond between N at the 3-position and C at the 2-position of the imidazole skeleton is a double bond, and the bond between C at the 2-position and N at the 1-position is a double bond when the bond between N at the 3-position and C at the 2-position is a single bond.

8. The antiviral agent according to claim 7, wherein R⁴ is 6-halogenopyridin-3-yl.

9. The antiviral agent according to claim 7 or 8, wherein R¹ is pyridin-2-yl having halogen-substituted C₁-C₆ alkyl at the 3-position and halogen at the 5-position.

10. The antiviral agent according to any one of claims 7 to 9, wherein A is methylene.

11. The antiviral agent according to any one of claims 7 to 10, wherein R¹ is 3-fluoro-5-(trifluoromethyl)pyridin-2-yl, and R⁴ is 6-fluoropyridin-3-yl.

12. The antiviral agent according to any one of claims 7 to 11, wherein the compound is any of the following compounds: 2-[[4-(5-bromo-2-(6-fluoropyridin-3-yl)-1H-imidazol-4-yl)-3-methoxyphenoxy]methyl]-3-fluoro-5-(trifluoromethyl)pyridine 2-[[4-(5-chloro-2-(6-fluoropyridin-3-yl)-1H-imidazol-4-yl)-3-methoxyphenoxy]methyl]-3-fluoro-5-(trifluoromethyl)pyridine 2-[[3-chloro-4-(5-chloro-2-(6-fluoropyridin-3-yl)-1H-imidazol-4-yl)phenoxy]methyl]-3-fluoro-5-(trifluoromethyl)pyridine

13. The antiviral agent according to any one of claims 7 to 12, wherein the virus is coronavirus.
